# EUROPÄISCHE PATENTANMELDUNG

(11) **EP 1 672 006 A1**
(43) Veröffentlichungstag der Anmeldung: **21.06.2006**
(21) Anmeldenummer: 04029522.2
(22) Anmeldetag: 14.12.2004
(51) Int. Cl.: C08J 3/03

(54) **Wässrige Dispersionen von Harnstoffgruppen enthaltenden Polyorganosiloxanen**

(71) Anmelder: Ciba Spezialitätenchemie Pfersee GmbH, 86462 Langweid a.L. (DE)
(72) Erfinder: Niederstadt, Rule, 86157 Augsburg (DE); Chrobaczek, Harald, Dr., 86153 Augsburg (DE); Angele, Theodor, 86637 Wertingen (DE); Lüdemann, Simpert, 86399 Bobingen (DE)

(57) **Zusammenfassung**

Harnstoffgruppen enthaltende Polyorganosiloxane lassen sich in Form wässriger Dispersionen erhalten, indem man sie zuerst in organischem Lösungsmittel löst, dann diese Lösung mit Wasser unter Mitverwendung von Hilfsmitteln dispergiert, die Mischung einer Homogenisierung unterwirft und anschließend das organische Lösungsmittel mindestens teilweise entfernt. Die so hergestellten wässrigen Dispersionen eignen sich als Zusatz zu Waschmitteln, Wäscheweichspülern, Haarpflegemitteln, Shampoos und für die Textilausrüstung.

## Beschreibung

Die Erfindung betrifft wässrige Dispersionen, welche Polysiloxane mit Harnstoffgruppen enthalten und mittels eines speziellen Verfahrens hergestellt werden können. Sie betrifft ferner die Verwendung solcher Dispersionen.

Wässrige Dispersionen von Polyorganosiloxanen finden in Form von Emulsionen ("Silikonemulsionen") flüssiger bzw. fließfähiger Polysiloxane vielfach Anwendung, so z.B. in der Textilausrüstung bzw. Textilveredelung, wobei textile Flächengebilde wie Gewebe mit diesen Emulsionen behandelt werden. Der Einsatz von solchen Polyorganosiloxanen erfolgt häufig in Form wässriger Emulsionen, weil hierbei Kosten- und Umweltvorteile gegenüber Einsatz als Lösung in organischen Lösungsmitteln resultieren.

Wässrige Dispersionen von speziellen Polyorganosiloxanen, ihre Herstellung und ihre Verwendung sind unter anderem in der WO 88/08436 beschrieben.
Bekannt sind aus dem Stand der Technik auch Harnstoffgruppen enthaltende Polyorganosiloxane, z.B. aus der EP-A 455 585.
Die DE-A 1 570 576 beschreibt Emulsionen von Polyorganosiloxanen, welche salzartige Gruppen aufweisen. Aus der EP-A 1 226 813 geht die Verwendung von Dispersionen für Haarpflege hervor, welche spezielle Polyorganoxiloxane enthalten.
Die Herstellung von Silikonemulsionen ist ferner beschrieben in der DE-A 197 22 403 und in der DE-Patentschrift Nr. 900 018.

Es ist zwar aus dem Stand der Technik eine Vielzahl von Methoden bekannt, wie wässrige Emulsionen von solchen Polysiloxanen hergestellt werden können, die keine Harnstoffgruppen enthalten. So können beispielsweise bei Raumtemperatur flüssige Polyorganosiloxane auf direkte Art in stabile wässrige Dispersionen überführt werden, indem man diese Siloxane mit Dispergator vermischt und die Mischung anschließend in Wasser dispergiert oder indem man zuerst eine Mischung aus Wasser und Dispergator herstellt und das Polysiloxan in dieser Mischung dispergiert. Solche wässrigen Dispersionen können zu den unterschiedlichsten Zwecken verwendet werden. Jedoch hat sich herausgestellt, dass die Mehrheit der aus dem Stand der Technik bekannten Methoden zur direkten Dispergierung von Polyorganosiloxanen in Wasser nicht geeignet ist, Harnstoffgruppen enthaltende Polyorganosiloxane in stabile wässrige Dispersionen zu überführen. Solche Polyorganosiloxane sind nämlich in vielen Fällen bei Raumtemperatur nicht flüssig und nicht fließfähig, sondern liegen als thermoplastische Polymere vor, deren direkte Dispergierung in Wasser nach herkömmlichen Verfahren auf beträchtliche Schwierigkeiten stößt oder überhaupt nicht möglich ist. Andererseits besitzen Harnstoffgruppen enthaltende Polyorganosiloxane vorteilhafte Eigenschaften, sodaß es wünschenswert ist, sie für manche Einsatzgebiete zu verwenden und zwar möglichst in Form wässriger Dispersionen.

Der vorliegenden Erfindung lag die Aufgabe zugrunde, wässrige Dispersionen von Harnstoffgruppen enthaltenden Polyorganosiloxanen zur Verfügung zu stellen, die nach einem Verfahren herstellbar sind, das es gestattet, stabile wässrige Dispersionen auch von bei Raumtemperatur nicht-flüssigen und nicht-fließfähigen Harnstoffgruppen enthaltenden Polyorganosiloxanen zu erhalten. Ferner bestand die Aufgabe darin, solche Dispersionen der genannten Art zur Verfügung zu stellen, die sich hervorragend für die Verwendung in Waschmitteln, Wäscheweichspülmitteln, Haarwaschmitteln, Haarpflegemitteln und für die Ausrüstung/Veredelung von Textilien eignen.

Die Aufgabe wurde gelöst durch eine wässrige Dispersion, welche hergestellt werden kann durch folgende, nacheinander ablaufende Verfahrensschritte:
a) Herstellen einer Lösung eines Harnstoffgruppen enthaltenden Polyorganosiloxans oder eines Gemischs solcher Polyorganosiloxane in einem wasserfreien organischen Lösungsmittel, von dem bei Raumtemperatur nicht mehr als 5 g in 100 g Wasser löslich sind,
b) Vermischen der bei Schritt a) erhaltenen Lösung mit Wasser unter Verwendung eines Hilfsmittels, das ausgewählt ist aus einem oder mehreren Dispergatoren, Polyvinylalkohol (PVA) und Guarmehl (GuM) und Mischungen von 2 oder mehr dieser Produkte.
c) Homogenisieren der bei Schritt b) erhaltenen Mischung, vorzugsweise mittels Hochdruckhomogenisierung,
d) Entfernen von 90 % bis 100 % des bei Schritt a) verwendeten organischen Lösungsmittels.

Die erfindungsgemäßen Dispersionen eignen sich ausgezeichnet als Zusatz für Waschmittel und für Wäscheweichspülmittel. Sie können hierzu herkömmlichen Waschmitteln bzw. herkömmlichen Wäscheweichspülmitteln zugesetzt werden, die beim Endverbraucher, z.B. im Haushalt, zur Anwendung kommen.
Es hat sich herausgestellt, dass sich durch die oben genannte erfindungsgemäße Verwendung der Dispersionen als "fabric softener" gewaschene Artikel erhalten lassen, z.B. Textilien, welche besonders vorteilhafte Eigenschaften aufweisen, wie gute Knittererholungseigenschaften in Verbindung mit angenehm weichem Griff, also Eigenschaften, welche unter den Begriff "appearance after washing" fallen. Unter anderem können Celluloseartikel, welche vollständig oder zu einem erheblichen Teil aus Cellulosefasern bestehen, so behandelt werden.
Wenn erfindungsgemäße Dispersionen üblichen Waschmitteln oder Wäscheweichspülmitteln zugesetzt werden, so erfolgt dies vorzugsweise in einer solchen Menge, dass in dem Endprodukt 0,1 bis 70 Gew % an Harnstoffgruppen enthaltendem Polyorganosiloxan vorliegen, vorzugsweise 1 bis 50 Gew%.

Eine weitere bevorzugte Verwendung erfindungsgemäßer Dispersionen besteht darin, dass sie herkömmlichen Haarpflegemitteln oder Haarwaschmitteln wie Shampoos zugesetzt werden. Dem Haar bzw. der Frisur lässt sich hierdurch eine gute Formbeständigkeit verleihen.

Ferner lassen sich erfindungsgemäße Dispersionen auch für die Behandlung textiler Flächengebilde, vorzugsweise zur Behandlung von Geweben verwenden. Eine solche Behandlung kann im Rahmen der Ausrüstung oder Veredelung von Textilien erfolgen, welche zeitlich nach üblichen Vorbehandlungs- und Färbeprozessen, aber noch vor der Konfektionierung durchgeführt wird. Den Textilien lassen sich hierdurch gute Formbeständigkeits- bzw. Knittereigenschaften verleihen. Den erfindungsgemäßen Dispersionen können hierbei übliche Produkte zugesetzt werden, wie sie zur Textilausrüstung bekannt sind, wie z.B. Cellulosevernetzer, Flammschutzmittel, usw. Erfindungsgemäße Dispersionen sind gut geeignet zur Behandlung von Geweben, die ganz oder zu einem erheblichen Teil aus Cellulosefasern bestehen.
Die Anwendung erfindungsgemäßer Dispersionen für Textilbehandlung kann nach üblichen, dem Fachmann bekannten, Methoden erfolgen, z.B. mittels Foulardierung.

Wenn erfindungsgemäße Dispersionen für die genannte Ausrüstung von textilen Flächengebilden verwendet werden sollen, ist es häufig von Vorteil, den Dispersionen vor ihrer Anwendung ein Polyorganosiloxan zuzusetzen, welches eine oder mehrere Epoxygruppen im Molekül enthält. Solche Epoxygruppen enthaltende Polyorganosiloxane sind auf dem Markt erhältliche Produkte. Beispiele hierfür sind die Produkte Wacker IM 35, Wacker Siliconöl M643, Wacker SLM 441112/A, Wacker OG-144 dest. und Wacker Epoxy-Öl OG 268 der Firma Wacker-Chemie, GmbH, DE.

Das Epoxygruppen enthaltende Polyorganosiloxan wird hierbei der erfindungsgemäßen Dispersion vorzugsweise in solchen Mengen zugesetzt, dass 6 bis 12 g Polysiloxan, welches Epoxygruppen enthält, pro 100 g erfindungsgemäßer Dispersion eingesetzt werden. Dieser Bereich von 6 bis 12 g ist auf 100 %iges, unverdünntes, Epoxygruppen enthaltendes Polyorganosiloxan bezogen. Dieses Polyorganosiloxan kann als unverdünntes Produkt zugesetzt werden oder in Form einer wässrigen Dispersion.
Die vorteilhafte Wirkung eines Zusatzes von Epoxygruppen enthaltendem Polyorganosiloxan besteht darin, dass dadurch den ausgerüsteten Textilien häufig weiter verbesserte Eigenschaften bezüglich "appearance after washing" verliehen werden, z.B. Verbesserung der Bügelfreiheit bzw. des Knitterverhaltens. Gut geeignet als Epoxygruppen enthaltende Polyorganosiloxane sind solche mit einer Epoxyzahl von 0,300 bis 0,450 (mol Epoxid/100 g).

Zur Herstellung erfindungsgemäßer Dispersionen verwendet man ein Polyorganosiloxan, das Harnstoffgruppen enthält. Es kann auch ein Gemisch solcher Polyorganosiloxane verwendet werden. Die Verwendung eines Gemischs solcher Siloxane ist der Normalfall, weil bei der Synthese dieser Polymeren Gemische von Molekülen mit unterschiedlichem Molekulargewicht anfallen. Diese Gemische können direkt zur Herstellung erfindungsgemäßer Dispersionen verwendet werden. Ein Harnstoffgruppen enthaltendes Polyorganosiloxan enthält also mindestens eine Einheit der Formel

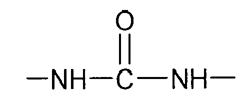

Selbstverständlich kann es auch mehrere solcher Einheiten enthalten.

Diese Einheiten können jeweils über eine Alkylengruppe an ein Siliciumatom gebunden sein, wobei diese Alkylengruppe durch eine oder mehrere sekundäre oder tertiäre Aminogruppen unterbrochen sein kann.

Harnstoffgruppen der Formel 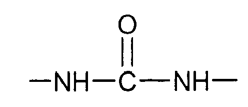 können an einem oder beiden Kettenenden des Polyorganosiloxans vorliegen. In diesem Fall weisen die Polyorganosiloxane an einem oder beiden Kettenenden vorzugsweise Einheiten der Formel (I) auf 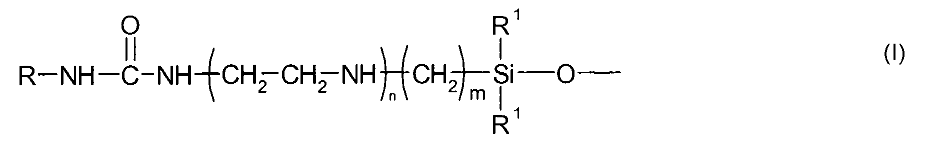 wobei m eine Zahl von 1 bis 4 ist und n den Wert 0 oder 1 besitzt,
die Reste R¹ unabhängig voneinander jeweils eine Alkylgruppe mit 1 bis 6 Kohlenstoffatomen bedeuten, vorzugsweise eine Methylgruppe, und wobei R für einen Alkylrest mit 1 bis 8 Kohlenstoffatomen oder für eine Phenylgruppe stehen kann, wobei diese Phenylgruppe durch eine oder mehrere Alkylgruppen mit jeweils 1 bis 4 Kohlenstoffatomen substituiert sein kann. Vorzugsweise jedoch steht der Rest R in Formel (I) für einen Rest der Formel (II)

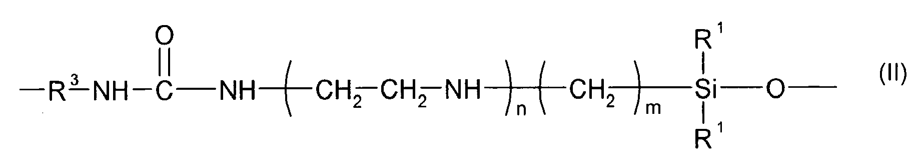

Hierbei ist der zweiwertige Rest R³ an das endständige Stickstoffatom von Formel (1) gebunden, (an das in Formel (1) der Rest R gebunden ist).
Bei dieser Ausführungsform sind zwei lineare Polyorganosiloxanketten an ihren Enden über eine Brücke verbunden, welche zwei Harnstoffeinheiten 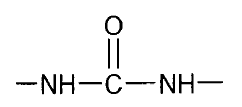 enthält, zwischen denen sich der zweiwertige Rest R³ befindet.
In Formel (II) besitzen m, n und R¹ die oben genannten Bedeutungen, R³ in Formel (II) steht für einen linearen oder verzweigten Alkylenrest mit 2 bis 10 Kohlenstoffatomen oder für einen zweiwertigen aromatischen Rest, wobei dieser aromatische Rest vorzugsweise ein Rest ist, der sich von Toluylendiisocyanat oder Diphenylmethandiisocyanat durch Entfernung der beiden NCO-Gruppen ableitet.

Ganz besonders gut geeignet für die Herstellung erfindungsgemäßer Dispersionen sind Harnstoffgruppen enthaltende Polyorganosiloxane, bei denen sich mindestens eine Harnstoffgruppe der Formel 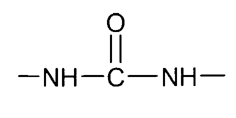 in einer Seitenkette des Polyorganosiloxans befindet. Solche bevorzugt verwendeten Polyorganosiloxane enthalten also eine oder mehrere Struktureinheiten der Formel (III) 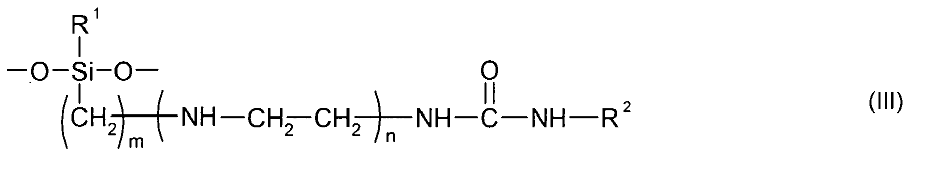 wobei R¹, m und n die oben genannten Bedeutungen besitzen und R² für einen Alkylrest mit 1 bis 8 Kohlenstoffatomen oder für eine Phenylgruppe stehen kann, wobei diese Phenylgruppe durch eine oder mehrere Alkylgruppen mit jeweils 1 bis 4 Kohlenstoffatomen substituiert sein kann.
Besonders bevorzugt ist es, wenn der Rest R² in Formel (III) für einen Rest der Formel (IV) steht, 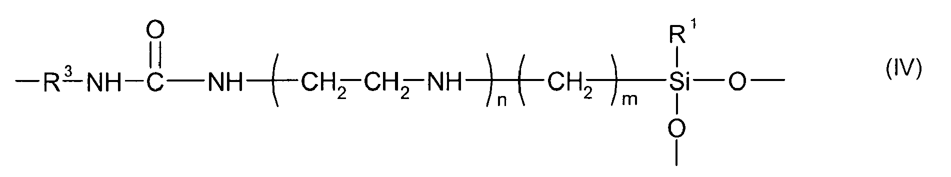 wobei der zweiwertige Rest R³ an das endständige Stickstoffatom von Formel (III) gebunden ist (an das in Formel (III) der Rest R² gebunden ist).
In Formel (IV) sind also zwei Polyorganosiloxanketten an ihren Seitenketten über eine oder mehrere Brücken miteinander verbunden, die Harnstoffgruppen enthalten. In Formel (IV) besitzen m, n, R¹ und R³ die oben genannten Bedeutungen.

Die beschriebenen Polyorganosiloxane mit Harnstoffgruppen lassen sich vorzugsweise dadurch herstellen, daß man Polyorganosiloxane, die einen oder mehrere Reste mit jeweils einer endständigen primären Aminogruppe enthalten, mit Mono- oder Diisocyanaten umsetzt. Dabei bilden sich durch Addition jeweils einer -NH₂-Gruppe an eine -NCO-Gruppe eine oder mehrere Harnstoffeinheiten

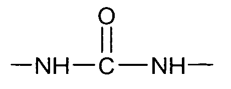

Es können für diese Umsetzung eine oder mehrere Arten von Polyorganosiloxanen und/oder eine oder mehrere Arten von Mono- oder Diisocyanaten verwendet werden. Auch Gemische aus Mono- und Diisocyanaten können eingesetzt werden.
Die Umsetzung der primären Aminogruppen des Polyorganosiloxans mit Isocyanatgruppen kann unter bekannten Bedingungen erfolgen, wie sie dem Chemiker geläufig sind. Verwendet man für die genannte Umsetzung Polyorganosiloxane, welche Reste der Formel 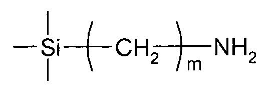 und keine sekundären oder tertiären Aminogruppen enthalten, so entstehen Polyorganosiloxane, welche Einheiten der Formel (I) oder der Formel (III) enthalten, bei denen n = 0 ist. Produkte mit n = 1 lassen sich dagegen herstellen, indem man Polyorganosiloxane mit Einheiten der Formel (V) 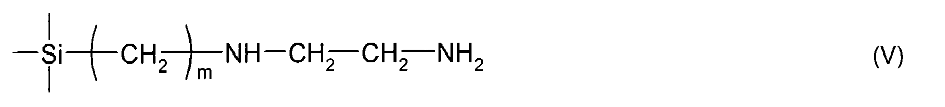 mit Isocyanaten umsetzt. Harnstoffgruppen enthaltende Polyorganosiloxane, in denen die Einheiten, die 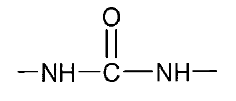 enthalten, in Seitenketten des Polymeren anwesend sind, lassen sich ausgehend von aminofunktionellen Polyorganosiloxanen herstellen, welche eine oder mehrere primäre Aminogruppen in Seitenketten enthalten.
Setzt man die aminofunktionellen Polyorganosiloxane mit Monoisocyanaten RNCO bzw. R²NCO um, so können Polymere erhalten werden, in welchen Struktureinheiten der Formel (I) oder (III) vorliegen, bei denen R bzw. R² für einen Alkylrest mit 1 bis 8 Kohlenstoffatomen oder für eine, ggf. substituierte, Phenylgruppe steht. Bei dieser Umsetzung verwendet man also Monoisocyanate der Formel RNCO bzw. R²NCO, in denen R bzw. R² die zuletzt genannte Bedeutung besitzt.
Besonders bevorzugt jedoch verwendet man zur Herstellung erfindungsgemäßer Dispersionen Polyorganosiloxane, die durch Umsetzung von aminofunktionellen Polyorganosiloxanen mit Diisocyanaten der Formel OCN-R³-NCO entstehen. In den hierbei gebildeten Harnstoffgruppen enthaltenden Polyorganosiloxanen sind zwei Polyorganosiloxanketten entweder an ihren Enden oder an ihren Seitenketten mittels Brücken verbunden, welche Harnstoffgruppierungen enthalten.
Als Diisocyanate können hierzu beispielsweise verwendet werden:
Hexamethylendiisocyanat, Isophorondiisocyanat, Toluylendiisocyanat oder Diphenylmethan-4,4'-diisocyanat.
Die aminofunktionellen Polyorganosiloxane, welche mit Diisocyanaten umgesetzt werden, sind bevorzugt Polydimethylsiloxane, in denen eine oder mehrere Methylgruppen durch Reste der Formel (V1) 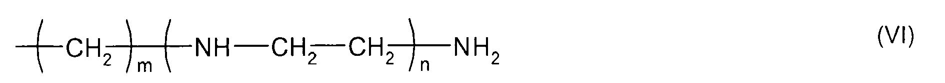 substituiert sind. Solche aminofunktionellen Polysiloxane sind auf dem Markt erhältlich, z.B. von den Firmen Wacker oder Dow Corning.
Geeignete, Harnstoffgruppen enthaltende, Polyorganosiloxane und ihre Herstellung sind beschrieben in der EP-A 455 585, der US 3 878 168 und der WO 96/34030.
Gut geeignet für die Herstellung erfindungsgemäßer Dispersionen sind Harnstoffgruppen enthaltende Polyorganosiloxane die bei Raumtemperatur nicht flüssig und nicht fließfähig sind und die einen Erweichungspunkt oder -bereich oberhalb von 50°C besitzen, bzw. thermoplastische, Harnstoffgruppen enthaltende, Polysiloxane. Vor allem bei solchen Siloxanen ist die Herstellung stabiler wässriger Dispersionen durch direkte Dispergierung der Polymeren in Wasser schwierig oder nicht durchführbar. Andererseits aber besitzen diese, Harnstoffgruppen enthaltenden, Polyorganosiloxane den Vorteil, dass mit ihnen ausgerüstete Textilien besonders gute Formstabilitäten nach Waschvorgängen aufweisen.

Vorzugsweise werden zur Herstellung erfindungsgemäßer Dispersionen solche Polyorganosiloxane verwendet, in denen an die Siliciumatome keine anderen Reste gebunden sind als Harnstoffgruppen oder Urethangruppen enthaltende Reste oder Alkylgruppen mit 1 bis 6 Kohlenstoffatomen oder unsubstituierte Phenylreste. Alle Endgruppen der Polyorganosiloxane, in welchen weder Harnstoffgruppen noch Urethangruppen vorliegen, sind also vorzugsweise Gruppen der Formel

(R⁴)₃Si―O―

wobei alle Reste R⁴ unabhängig voneinander für eine Alkylgruppe mit 1 bis 6 Kohlenstoffatomen oder für den Phenylrest C₆H₅ stehen.

Harnstoffgruppen enthaltende Polyorganosiloxane, welche zur Herstellung erfindungsgemäßer Dispersionen verwendet werden können, sind auf dem Markt erhältlich.

Die Harnstoffgruppen enthaltenden Polyorganosiloxane können neben Harnstoffeinheiten noch zusätzlich eine oder mehrere Urethangruppen der Formel 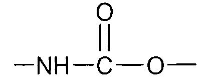 enthalten, in denen das endständige Sauerstoffatom an ein Kohlenstoffatom gebunden ist. Solche Urethangruppen können gebildet werden, indem man bei der oben beschriebenen Umsetzung von aminofunktionellen Polyorganosiloxanen mit Diisocyanaten Polyorganosiloxane verwendet, die zusätzlich Hydroxygruppen enthalten.

Erfindungsgemäße wässrige Dispersionen können durch das nachfolgend beschriebene Verfahren hergestellt werden, wobei die Reihenfolge a - b - c - d der Verfahrensschritte eingehalten werden muß.

### Schritt a):

Bei diesem Verfahrensschritt wird eine Lösung des verwendeten Polyorganosiloxans oder des Gemischs aus Polyorganosiloxanen in einem organischen Lösungsmittel hergestellt. Es kann auch ein Gemisch von zwei oder mehr organischen Lösungsmitteln verwendet werden. Solche Gemische werden ebenfalls nachfolgend als "organisches Lösungsmittel" bezeichnet. Gegebenenfalls erfolgt die Herstellung der Lösung bei erhöhter Temperatur. Entweder verwendet man zur Herstellung der Lösung in Schritt a) keine anderen Siloxane oder Polyorganosiloxane als Harnstoffgruppen enthaltende Polyorganosiloxane der oben beschriebenen Art. Oder aber man verwendet bei der Herstellung der Lösung gemäß Schritt a) ein oder mehrere weitere Polyorganosiloxane, welche keine Harnstoffgruppen enthalten. Gut geeignet hierfür sind bekannte, Aminogruppen enthaltende, Polyorganosiloxane, die auf dem Markt erhältlich sind. Die Menge an solchen zusätzlich verwendeten Polyorganosiloxanen beträgt vorzugsweise 1 bis 50 g pro 100 g an Harnstoffgruppen enthaltendem Polyorganosiloxan.
Vorteilhaft ist es, wenn die in Schritt a) hergestellte Lösung 5 bis 50 Gew% Polyorganosiloxan enthält, vorzugsweise 10 bis 40 Gew%. Diese Mengenangabe bezieht sich auf die Summe aller anwesenden Polyorganosiloxane
Auch nach Durchführung der Verfahrensschritte a) bis d) können den erfindungsgemäßen Dispersionen, bevor diese weiterverarbeitet werden, andere Polysiloxane zugesetzt werden, z.B. handelsübliche flüssige aminofunktionelle Polyorganosiloxane oder wässrige Dispersionen solcher Siloxane.

Das für Schritt a) verwendete organische Lösungsmittel muß wasserfrei sein. Dies bedeutet nicht, dass es überhaupt kein Wasser enthalten darf, jedoch darf der Wassergehalt dieses Lösungsmittels nicht höher sein als 2 Gew%. Natürlich soll auch das verwendete Polyorganosiloxan nicht mehr als etwa 2 Gew% Wasser enthalten.
Darüber hinaus darf das verwendete Lösungsmittel nur eine geringe Wasserlöslichkeit besitzen. Bei Raumtemperatur darf diese Löslichkeit nicht größer sein als 5 g Lösungsmittel in 100 g Wasser.
Als Lösungsmittel geeignet sind höhere Alkohole, Ester, Ketone, z.B. Dialkylketone aber auch aromatische Kohlenwasserstoffe wie Toluol oder Xylol, sofern sie die oben genannten Bedingungen erfüllen und das verwendete Polyorganosiloxan oder -gemisch in der gewünschten Konzentration in dem Lösungsmittel löslich ist, wobei zum Lösen gegebenenfalls erhöhte Temperatur erforderlich sein kann.
Bevorzugt werden im Hinblick auf Verfahrensschritt d) (Entfernen von mindestens 90 % des Lösungsmittels) solche Lösungsmittel verwendet, die sich gut aus den bei Schritt c) hergestellten Dispersionen entfernen lassen. Eine einfache Methode hierzu ist Destillation, gegebenenfalls unter vermindertem Druck. Zu den Lösungsmitteln, die sich auf diese Art zu mindestens 90 % entfernen lassen, gehören Lösungsmittel mit einem Siedepunkt von weniger als 100°C bei Raumtemperatur, Lösungsmittel, die mit Wasser ein Azeotrop bilden oder Lösungsmittel, die bei vermindertem Druck einen höheren Dampfdruck aufweisen als Wasser. Besonders gut geeignet als Lösungsmittel sind Methyl-isobutyl-keton und n-Butyl-acetat; beide lassen sich in Schritt d) gut als Azeotrop mit Wasser destillativ entfernen

### Schritt b):

In Verfahrensschritt b) wird die nach Schritt a) erhaltene Lösung mit Wasser vermischt. Bei diesem Mischvorgang wird ein Hilfsmittel, wie unten beschrieben, zusätzlich verwendet.
Das Vermischen kann im Normalfall bei Raumtemperatur erfolgen. Gegebenenfalls besitzt die Lösung des Polyorganosiloxans oder das Wasser oder beide eine erhöhte Temperatur. Es empfiehlt sich, zur Unterstützung des Mischvorgangs, das Gemisch mechanisch zu rühren.

Das Vermischen erfolgt vorzugsweise in solchen Mengenverhältnissen, dass die fertige, nach Durchführung von Schritt d) erhaltene wässrige Dispersion 5 bis 70 Gew%, insbesondere 10 bis 50 Gew%, an Harnstoffgruppen enthaltenden Polyorganosiloxanen enthält. Ferner enthält die fertige erfindungsgemäße Dispersion vorzugsweise 0,5 bis 10 Gew% an einem Hilfsmittel der unten beschriebenen Art.
Dieses Hilfsmittel kann vor dem Vermischen der Lösung des Polyorganosiloxans mit Wasser dieser Lösung zugesetzt werden. Bevorzugt ist es jedoch, das Hilfsmittel dem Wasser zuzusetzen, bevor es mit der bei Schritt a) erhaltenen Lösung vermischt wird.

In Schritt b) wird ein Hilfsmittel mitverwendet. Dieses ist ausgewählt aus einem oder mehreren Dispergatoren, Polyvinylalkohol (PVA) und Guarmehl (GuM). Auch Mischungen sind geeignet, welche einen oder mehrere Dispergatoren und zusätzlich entweder PVA oder GuM enthalten oder die zusätzlich zu Dispergator(en) sowohl PVA als auch GuM enthalten. Daneben kann in einer Reihe von Fällen auf Dispergatoren ganz verzichtet werden. In diesen Fällen wird nur PVA oder nur GuM oder eine Mischung PVA/GuM verwendet.
In jedem der genannten Fälle wird die Gesamtmenge an Hilfsmittel bevorzugt so gewählt, dass die nach Schritt d) erhaltene erfindungsgemäße Dispersion 0,5 bis 10 Gew% des Hilfsmittels enthält, unabhängig davon, ob das Hilfsmittel nur aus einem einzigen Produkt aus der Gruppe Dispergator/PVA/GuM besteht oder aus einer Mischung, welche 2 oder mehr solcher Produkte enthält.

Als Dispergatoren geeignet sind bekannte handelsübliche oberflächenaktive Produkte. Bevorzugt werden nichtionische oder kationische Dispergatoren verwendet oder ein Gemisch von Dispergatoren, die aus nichtionischen und kationischen Dispergatoren ausgewählt sind. Die Menge an Dispergator oder -gemisch kann in dem Bereich liegen, wie er dem Fachmann auf dem Gebiet der Silikonemulsionen bekannt ist. Vorzugsweise verwendet man solche Mengen, dass die fertige erfindungsgemäße Dispersion, die nach Durchführung von Schritt d) erhalten wird, 0,5 bis 10 Gew% an Dispergator oder Dispergatorgemisch enthält. Diese letztere Aussage gilt dann, wenn das verwendete Hilfsmittel ausschließlich aus einem oder mehreren Dispergatoren besteht.
Als nichtionische Dispergatoren sind bekannte handelsübliche oberflächenaktive Produkte geeignet wie z.B. ethoxilierte Alkohole, ethoxilierte Fettsäuren oder ethoxilierte Fettamine, jeweils mit linearen oder verzweigten, gesättigten oder ungesättigten Kohlenwasserstoffresten, die 10 - 24 Kohlenstoffatome enthalten. Beispiele hierfür sind ethoxilierte Isotridecylalkohole und ethoxilierte Stearinsäure oder Ölsäure mit jeweils 6 bis 12 Polyoxyethyleneinheiten.
Als kationaktive Dispergatoren kommen insbesondere Ammoniumsalze der Formel (VII) in Betracht. 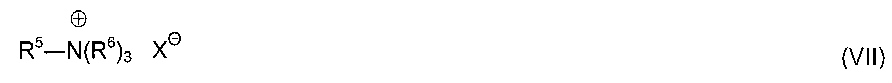 wobei R⁵ für einen linearen oder verzweigten, gesättigten oder ungesättigten Kohlenwasserstoffrest mit 8 bis 24 Kohlenstoffatomen steht, alle Reste R⁶ unabhängig für Wasserstoff oder einen Alkylrest mit 1 bis 7 Kohlenstoffatomen stehen, der durch eine Hydroxygruppe substituiert sein kann, oder für einen Rest R⁵ stehen. Vorzugsweise stehen zwei oder drei Reste R⁶ für eine Methyl-, eine Ethyl- oder eine 2-Hydroxyethylgruppe. Gegebenenfalls können eine oder mehrere der Reste R⁶ auch für einen Polyoxyethylenrest mit 6 bis 18 Polyoxyethyleneinheiten stehen.

Das Hilfsmittel kann auch PVA neben Dispergator(en) und/oder GuM enthalten oder nur aus PVA bestehen. In diesem Fall verwendet man vorzugsweise PVA mit einem mittleren Molgewicht im Bereich von 25.000 bis 100.000. Ebenso kann das Hilfsmittel neben Dispergator(en) und/oder PVA noch GuM enthalten oder nur aus GuM bestehen. Bevorzugt verwendet man hierzu GuM, das durch folgende Daten charakterisiert wird: Viskosität einer 1 % Lösung bei 25°C: 3,5 - 3,7 Pa.s (3500 - 3700 cP) mittlere Teilchengröße: ca. 75 · 10⁻⁶ m. GuM ist im Handel erhältlich, z. B. von der Firma Worlée-Chemie GmbH, DE. Guarmehl (GuM) ist ein aus der Literatur bekanntes Produkt, siehe z.B. "Römpp Chemie Lexikon", Georg Thieme Verlag Stuttgart, New York, 9. Auflage, 1990, Seite 1666.
PVA und GuM können bei erfindungsgemäßen Dispersionen eine erhöhte Stabilität der wässrigen Dispersion bewirken, indem sie als Schutzkolloid fungieren.

Eine besonders bevorzugte Ausführungsform erfindungsgemäßer Dispersionen besteht darin, dass bei ihrer Herstellung in Schritt b) als Hilfsmittel ein Gemisch verwendet wird, das einen oder mehrere kationaktive Dispergatoren und zusätzlich PVA und/oder GuM enthält. Als kationaktive Dispergatoren sind hierbei die oben bereits genannten Produkte gut geeignet.

### Schritt c):

Um stabile wässrige Dispersionen der Harnstoffgruppen enthaltenden Polyorganosiloxane zu erhalten, ist es erforderlich, das nach Schritt c) erhaltene Gemisch aus Polysiloxan, Hilfsmittel und Wasser einer Homogenisierung zu unterwerfen. Diese Homogenisierung kann je nach Art und Menge der verwendeten Produkte gegebenenfalls bei Raumtemperatur erfolgen. Vorzugsweise jedoch wird sie bei erhöhter Temperatur durchgeführt, z.B. im Bereich von 40 - 80°C. Vorteilhaft ist es ferner, die Homogenisierung in Form einer Hochdruckhomogenisierung durchzuführen.
Die Hochdruckhomogenisierung wird vorzugsweise bei einem Druck im Bereich von 50 bis 1000 bar durchgeführt, ein besonders günstiger Bereich sind 50 bis 350 bar. Der Druck lässt sich bei einer Reihe bekannter Apparaturen von der unten beschriebenen Art gezielt einstellen und steuern.
Angaben zu Vorrichtungen und Verfahren für Hochdruckhomogenisierung können der WO 99/15263 entnommen werden.
Zur Durchführung der Hochdruckhomogenisierung gemäß Verfahrensschritt c) eignen sich Gaulin-Geräte der Firma Manton-Gaulin Corp. USA bzw. der Firma APV-Deutschland GmbH, Lübeck, DE, z.B. die Type "Gaulin-Homogenisator LAB 60-7 TBS" oder die MC-Typen dieser Firma. Ferner geeignet sind Geräte der "Microfluidizer"-Serie der Firma Microfluidics, USA.

### Schritt d):

Der letzte Schritt des Verfahrens, durch das erfindungsgemäße Dispersionen hergestellt werden können, besteht darin, 90 % bis 100 % der Menge des für Schritt a) verwendeten Lösungsmittels zu entfernen. Für eine Reihe von Anwendungszwecken, z.B. für die Verwendung in Waschmitteln oder Wäscheweichspülmitteln oder die Verwendung für Haarwasch-/Haarpflegemittel ist es zweckmäßig, das Lösungsmittel vollständig oder nahezu vollständig zu entfernen. In manchen Fällen jedoch kann es genügen oder sogar erwünscht sein, nur einen Teil des Lösungsmittels zu entfernen, z.B. beim Einsatz für Beschichtungszwecke wie Beschichtung von Geweben. Produkte die nach dem in der vorliegenden Patentanmeldung beschriebenen Verfahren hergestellt werden, sind also wässrige Dispersionen im Sinne der vorliegenden Erfindung, selbst wenn sie noch bis zu 10 % derjenigen Menge an Lösungsmittel enthalten, die für Schritt a) verwendet wurde.
Die teilweise oder vollständige Entfernung des Lösungsmittels kann nach bekannten Methoden erfolgen. Im Normalfall ist eine Destillation zweckmäßig. Durch Auswahl geeigneter Lösungsmitteleigenschaften kann, wie oben beschrieben, die destillative Abtrennung ermöglicht werden.

Die Erfindung wird nachfolgend durch Ausführungsbeispiele veranschaulicht.

### Beispiel 1 (erfindungsgemäß)

### Herstellung einer erfindungsgemäßen Dispersion:

100 g eines Polydimethylsiloxans, das Harnstoffgruppen enthält (TPS bzw. TPSE 140 der Firma Wacker GmbH, DE) wurden in 300 g Methyl-isobutylketon (MIBK) bei etwa 80°C gelöst. Zu dieser Lösung wurden 600 g einer wässrigen Dispergatorlösung zugegeben. Der pH-Wert des erhaltenen Gemischs wurde durch Zugabe 60 %iger Essigsäure auf einen Wert von ca. 3,3 bei 60°C eingestellt.
Die wässrige Dispergatorlösung enthielt etwa 550 g Wasser, 20 g einer Mischung aus einem kationaktiven Dispergator (quartäres Ammoniumsalz), und einem nichtionogenen Dispergator sowie 20 g 1.2-Propylenglykol.
Anschließend wurde gerührt, wobei eine Voremulsion gebildet wurde; diese wurde einer Hochdruckemulgierung bei etwa 50°C unterworfen, wobei sich eine homogene Dispersion bildete. Aus dieser wurde MIBK durch Destillation unter vermindertem Druck praktisch quantitativ (als Azeotrop mit Wasser) entfernt. Es resultierte eine leicht opaleszente Dispersion mit einem Gehalt an Polysiloxan + Dispergator von etwa 29 Gew%.

### Beispiel 2 (erfindungsgemäß)

Es wurde eine Lösung eines Harnstoffeinheiten enthaltenden Polydimethylsiloxans in MIBK wie in Beispiel 1 hergestellt und weiterverarbeitet mit dem Unterschied, dass an Stelle von 20 g eines Dispergatorgemischs 22 g eines Gemischs aus kationaktivem Dispergator und Polyvinylalkohol (PVA) (Polyviol 25/140 der Firma Wacker GmbH, DE) hinzugefügt wurde.
Man erhielt eine opaleszente Dispersion mit etwa 24 Gew% Anteilen außer Wasser.

### Beispiel 3 (erfindungsgemäß)

Es wurde gearbeitet wie in Beispiel 2, wobei an Stelle von 22 g einer Mischung aus kationaktivem Dispergator und PVA jetzt 5 g einer Mischung aus kationaktivem Dispergator und Guarmehl (GuM) (WOGU 4401 der Firma Worlée, DE) verwendet wurden. Man erhielt eine leicht opaleszente Dispersion.

### Beispiel 4 (nicht erfindungsgemäßes Vergleichsbeispiel)

Es wurde versucht, das Harnstoffgruppen enthaltende Polydimethylsiloxan, das in den vorangegangenen Beispielen verwendet worden war, auf üblichem Weg in eine wässrige Dispersion zu überführen. Da dieses Siloxan von fester Konsistenz ist, wurde versucht, es als Feststoff aufzuschmelzen und dann zu dispergieren oder es als Feststoff einer wässrigen Dispergatorlösung zuzufügen und dann dieses Gemisch durch Erwärmen in eine wässrige Siloxandispersion zu überführen.
Beide Versuche schlugen fehl: es gelang nicht, auf diese Weise eine wässrige Dispersion des Polysiloxans zu erhalten.

### Beispiel 5

Mit den gemäß Beispielen 1 bis 3 erhaltenen Dispersionen, denen 4 Gew% eines handelsüblichen Cellulosevernetzers (KNITTEX® FEL der Firma Ciba Spezialitätenchemie Pfersee GmbH) zugesetzt worden war, wurden Gewebe aus 100 % Baumwolle mittels eines Foulardprozesses behandelt. Anschließend erfolgte Trocknung der Gewebe bei 110°C/10 min. und Kondensation 150°C/5 Minuten. Die Gewebe zeigten gutes Knitterverhalten, das dem von Geweben überlegen war, welche mit einer herkömmlichen Silikonemulsion, die Cellulosevernetzer enthielt, unter den gleichen Bedingungen behandelt worden waren.

### Beispiel 6

Beispiel 5 wurde wiederholt, wobei den erfindungsgemäßen Dispersionen gemäß Beispiel 1 bis 3 noch etwa 30 Gew.teile Epoxygruppen enthaltender Polysiloxandispersion (OG 144 der Firma Wacker GmbH, DE) pro 100 Gew.teilen erfindungsgemäßer Dispersion hinzugefügt wurden. Auch mit den 3 so hergestellten Dispersionen wurden ähnlich gute Gewebeeigenschaften erzielt wie in Beispiel 5.

## Patentansprüche

1. Wässrige Dispersion, herstellbar durch folgende, nacheinander ablaufende Verfahrensschritte:
a) Herstellen einer Lösung eines Harnstoffgruppen enthaltenden Polyorganosiloxans oder eines Gemischs solcher Polyorganosiloxane in einem wasserfreien organischen Lösungsmittel, von dem bei Raumtemperatur nicht mehr als 5 g in 100 g Wasser löslich sind,
b) Vermischen der bei Schritt a) erhaltenen Lösung mit Wasser unter Verwendung eines Hilfsmittels, das ausgewählt ist aus einem oder mehreren Dispergatoren, Polyvinylalkohol (PVA) und Guarmehl (GuM) und Mischungen von 2 oder mehr dieser Produkte.
c) Homogenisieren der bei Schritt b) erhaltenen Mischung, vorzugsweise mittels Hochdruckhomogenisierung,
d) Entfernen von 90 % bis 100 % des bei Schritt a) verwendeten organischen Lösungsmittels.

2. Dispersion nach Anspruch 1, **dadurch gekennzeichnet dass** in Schritt a) ein Harnstoffgruppen enthaltendes Polyorganosiloxan verwendet wird, das durch Umsetzung von einem eine oder mehrere primäre Aminogruppen enthaltenden Polyorganosiloxan mit Mono- oder Diisocyanat oder mit einem Gemisch aus Mono- und Diisocyanat hergestellt wurde.

3. Dispersion nach Anspruch 1 oder 2, **dadurch gekennzeichnet dass** die bei Schritt a) hergestellte Lösung 5 bis 50 Gew% Polyorganosiloxan enthält, vorzugsweise 10 bis 40 Gew%.

4. Dispersion nach einem oder mehreren der Ansprüche 1 bis 3, **dadurch gekennzeichnet, dass** in Schritt b) als Hilfsmittel ein nichtionischer oder ein kationischer Dispergator oder ein Gemisch solcher Dispergatoren verwendet wird.

5. Dispersion nach einem oder mehreren der Ansprüche 1 bis 4, **dadurch gekennzeichnet, dass** in Schritt b) als Hilfsmittel ein Gemisch verwendet wird, das einen oder mehrere kationaktive Dispergatoren und zusätzlich PVA und/oder GuM enthält.

6. Dispersion nach einem oder mehreren der Ansprüche 1 bis 5, **dadurch gekennzeichnet, dass** Schritt b) so durchgeführt wird, dass das Hilfsmittel dem Wasser vor dem Vermischen der bei Schritt a) erhaltenen Lösung mit Wasser zugesetzt wurde.

7. Dispersion nach einem oder mehreren der Ansprüche 1 bis 6, **dadurch gekennzeichnet, dass** Schritt c) bei einem Druck im Bereich von 50 bis 1000 bar durchgeführt wird, vorzugsweise bei 50 - 350 bar.

8. Dispersion nach einem oder mehreren der Ansprüche 1 bis 7, **dadurch gekennzeichnet, dass** sie 0,5 bis 10 Gew% eines Hilfsmittels enthält.

9. Dispersion nach einem oder mehreren der Ansprüche 1 bis 8, **dadurch gekennzeichnet, dass** sie 5 bis 70 Gew% an Harnstoffgruppen enthaltendem Polyorganosiloxan enthält, vorzugsweise 10 - 50 Gew%.

10. Verwendung einer Dispersion gemäß einem oder mehreren der Ansprüche 1 bis 9 als Zusatz zu Waschmitteln oder Weichspülmitteln.

11. Verwendung einer Dispersion gemäß einem oder mehreren der Ansprüche 1 bis 9 als Zusatz zu Haarpflegemitteln oder Haarwaschmitteln.

12. Verwendung einer Dispersion gemäß einem oder mehreren der Ansprüche 1 bis 9 zur Behandlung von textilen Flächengebilden im Rahmen der Textilausrüstung oder -veredelung.

13. Verwendung nach Anspruch 12, **dadurch gekennzeichnet, dass** der Dispersion vor der Behandlung der textilen Flächengebilde ein Polyorganosiloxan zugesetzt wurde, das eine oder mehrere Epoxygruppen im Molekül enthält.

14. Verwendung nach Anspruch 13, **dadurch gekennzeichnet, dass** das zugesetzte Polyorganosiloxan eine Epoxyzahl von 0,300 bis 0,450 (mol Epoxid/100 g) aufweist.
